# EUROPEAN PATENT SPECIFICATION

(11) **EP 1 656 897 B1**
(45) Date of publication and mention of the grant of the patent: **23.09.2009**
(21) Application number: 05292677.1
(22) Date of filing: 12.12.2002
(51) Int. Cl.: A61B 17/70

(54) **Implant for osseus anchoring with polyaxial head**
Knochenimplantat mit polyaxialem Kopf
Implant osseux avec tête polyaxiale

(30) Priority: 12.12.2001 FR 0116122
(43) Date of publication of application: 17.05.2006
(62) Divisional of application: 02788326.3
(73) Proprietor: LDR Medical, 10430 Rosières près Troyes (FR)
(72) Inventor: Beaurain, Jacques, 21910 Saulon La Chapelle (FR); Delecrin, Joel, 44120 Vertou (FR); Chataignier, Hervé, 25230 Boussieres (FR)
(74) Representative: Debay, Yves

(56) References cited:
- EP-A- 0 572 790
- DE-A- 19 605 640
- US-A- 5 536 268
- US-A- 5 733 286
- US-A- 6 063 090
- US-A1- 2001 001 119

## Description

The present invention relates to an implant providing osseous anchorage, for example in a vertebra for anchoring an osteosynthesis device. This implant comprises a head capable of receiving a bar linking a plurality of implants in different angular positions.

It is known to fix one or a plurality of implants into one or a plurality of osseous elements in order to connect to the skeleton a device implanted in the human or animal body, said implants being then used to fasten certain elements of said device. For maintaining or correcting the rachis in particular, use of an osteosynthesis device comprising one or a plurality of maintenance bars or plates positioned along the vertebral column and fixed to certain vertebrae by implants is well-known. These implants are fixed on the one hand to the bars and on the other to the vertebrae by an osseous anchoring means comprised of a hook having its support on a vertebra or of a threaded part screwed inside the vertebra, for example, at the pedicle. In the case of an osteosynthesis of the extreme vertebrae one or a plurality of implants can of course be securely fastened to adjacent bone, the sacrum for example.

The FR-A-2823095 patent discloses for this purpose an implant comprising an osseous anchoring part and a fixation head transversed by a channel where the bar is clamped. This document describes an implant, whose head comprises a lateral opening enabling the introduction of a bar with flat whereas a clamping screw has already been pre-installed in the superior part of said head. In order to assure satisfactory contact of the clamping screw on the flat whatever the angular position of said flat about the axis of the pin, the clamping screw is provided with a tiltable support surface mounted on a ball and socket joint.

By supporting itself on the inside shape of the channel under the effect of the clamping, the bar's position is thus definitively determined by the general position of the implant. At the time of installation of the bar in an implant already anchored in the rachis, if the bar is not in a position corresponding to that of the channel, the act of forcing its introduction can induce a certain stress in the bar.

On the one hand, said stress can then make the introduction of the bar into the implant difficult or render its clamping not very accurate. This problem is true more particularly when the bar has already been engaged in a first implant and is being introduced into a second implant. In order to reduce this stress, it is conceivable to anchor the implant according to the position of the bar, but this is not always possible to do nor easy to predict. It would also be conceivable to deform the bar, which could be a problem in the situation and plays against the requirement of a rigid bar to assure an effective hold. This stress can thus make manipulation delicate to execute, in particular in weale intrusion surgical procedures, for example video-assisted or laparoscopic procedures.

On the other hand, even if clamping enables stressing the bar to adopt a position or a shape corresponding to the implant, said stress will persist permanently over a very long time after the procedure. The fact that the bar is under permanent tension poses the risk of a mechanical effect directly on the rachis, for example causing pains or changing or disturbing the correction or the support sought by using the osteosynthesis device.

The document EP-A-0 572 790 discloses an implant according to the preamble of claim 1 capable of an adaptation to the orientation of the bar.

The document US-A-5 733 286 discloses a polyaxial screw and coupling element assembly and an orthopaedic rod implantation apparatus comprising a screw having a curvate head cooperating with a hemispherical insert for providing an articulation of a coupling element including a rod receiving channel, a threaded portion and an axial bore, said screw and insert being positioned in the axial bore of the coupling element, below the rod inserted in the channel. The assembly formed by the screw, the insert and the rod are crush locked by a locking means screwed in the threaded portion of the coupling element. This solution has the disadvantages that the rod receiving channel is open on its upper surface, thus only allowing the insertion of the rod from the upper surface of the coupling element and that the locking means contacts the rod with a flat surface, the clearance of the rod relative to the coupling element being thus restricted.

The object of the present invention is to eliminate at least one drawback of the prior art by providing an osseous anchorage implant capable of more adaptation to different orientations of the bar whereas the implant is already anchored in the osseous element.

This aim is achieved by an implant for osseus anchoring according to claim 1.

According to one feature, the fixation means comprise at least one element enabling, prior to the blocking of the fixation, a determined clearance in rotation around at least one second axis not parallel to the first axis and not parallel to the longitudinal axis of the bar.

According to one feature, the implant comprises on the one hand osseous anchoring means et on the other hand a fixation head bearing the fixation means, **characterized in that** the fixation head is traversed by at least one channel receiving the bar, and comprises clamping means capable of clamping the bar against one inside wall, called support wall, of the channel.

According to one feature, the channel opens at each side of the fixation head through apertures whose disposition and dimensions enable to the bar a determined clearance inside the channel in rotation at least around one axis that is substantially perpendicular to the axis of the channel and to the support direction of the clamping means.

According to one feature, the support wall comprises an element, called the moving baseplate, having a clearance in rotation at least around one axis not parallel to the axis of the channel and to the support direction of the clamping means on the bar, the clamping means comprising a face at the contact with the bar, called moving clamping face, having a determined clearance in rotation around one axis substantially parallel to the axis of rotation of the moving baseplate.

According to one feature, the moving clamping face of the clamping means is borne by a support head articulated at the end of the end of the clamping screw by a ball and socket connection.

According to one feature, the moving baseplate has a part in form of a sphere portion leaning by a complementary contact in a housing formed in the fixation head.

According to one feature, the osseous anchoring part is connected to the fixation head by an extremity, called rotation head, the fixation head being movable relating to this rotation head, at least prior the blocking of the clamping means.

According to one feature, the fixation head presents relating to the rotation head on the one hand an unlimited clearance in rotation around an axis, called head rotation axis, not parallel to the channel axis or to the longitudinal axis of the bar.

According to one feature, the fixation head presents relating to the rotation head a determined clearance in rotation around an axis, called head tilting axis, not parallel to the head rotation axis.

According to one feature, the clamping means bear the bar on a moving baseplate comprising sphere portion part, this sphere portion bearing in a complementary housing formed in the rotation head, the rotation head itself comprising an external rotation surface in form of a half sphere portion bellmouthed toward the fixation head, this external rotation surface being retained in a housing having a complementary shape prepared in the fixation head, the clamping of the clamping means inside the fixation head leading thus to a clamping and stopping of all of these surfaces being in contact.

According to one another feature, the moving base plate is in contact with the bar by a support face having a shape substantially complementary to the exterior shape of the bar, the moving baseplate having at least one formal irregularity co-operating with a formal irregularity borne by the rotation head to form a stop holding the support face of the baseplate turned towards the side of the bar.

According to one feature, the clamping means comprise a clamping screw inserted into a drilling traversing one edge of the channel opening and co-operating with said drilling to realize a support upon the bar.

According to one feature, the osseous anchoring means comprise either a threaded part capable of anchoring in an osseous element by co-operation with the osseous material of said osseous element, or a protruding part capable of anchoring at the surface of an osseous element by co-operating with at least one formal irregularity of said same surface.

According to one feature, the osseous anchoring means are realized by an elongated and threaded part, the axis of the channel being substantially perpendicular to the longitudinal axis of the osseous anchoring means.

According to one feature, the channel has a shape comprising two head to tail truncated cones facing each other with their minor baseplates and joined to each other directly or by means of a cylindrical part.

The invention together with its features and advantages will be clearer by reading the description thereafter with reference to the annexed drawings, wherein :
Figures 1, 2, and 3 represent in a top view an implant with moving head and osseous anchorage by threading in cross section longitudinal to the bar, and in side view along the axis of the bar and in a top view, respectively;
Figure 4 represents a partial side view of an implant not according to the invention with fixed head during introduction of the bar, in section transverse to the bar;
Figures 5 and 6 represent perspective views of an implant not according to the invention with fixed head and osseous anchorage through screw in longitudinal section, along the axis of the bar, and transverse to the bar, respectively;
Figure 7 represents a perspective view of an implant with fixed head not according to the invention but with osseous anchorage by hook, in longitudinal section along the axis of the bar and represented without its clamping means;
Figures 8 and 9 represent, respectively, a perspective view and a sectional view of an implant with fixed head not according to the invention but according to a second embodiment of the moving baseplate;
Figures 10 and 11 represent, respectively, a perspective view and a sectional view of an implant with fixed head not according to the invention but according to a third embodiment of the moving baseplate;
Figures 12 and 13 represent, respectively, a perspective view and a sectional view of an implant with fixed head not according to the invention but according to a fourth embodiment of the moving baseplate.

The invention described herein relates to an implant comprising on the one hand means for fixation of a bar and on the other hand means for anchoring to an osseous element.

In a general fashion, in the field of surgery, an implant is defined as an object intended for being implanted in the human or animal body and to remain there continuously after the surgical procedure, at least over a certain period of time. More precisely, one speaks of a prosthesis to designate a device realizing a function, for example a movement or an articulation. Although not comprising a prosthesis *per se*, it must be understood that the implant described herein can comprise part of a prosthesis device or can be used for fixation on such a device.

By way of example, the implant according to the invention is described herein in the form of an implant incorporated in an osteosynthesis device, such as used to hold, prop, or straighten the rachis. This function is thus assured by one or a plurality of rigid bars or similar elements, connecting a plurality of rachis elements with each other, such as the sacrum, the vertebrae or parts of vertebrae. In the same sense, such a bar can obviously be used also to connect another implant or prosthesis to the skeleton such as, for example, an artificial vertebra, an arthrodesis frame, or an intervertebral disc prosthesis.

In its part affixed to an osseous element, said bar is anchored to said osseous element by means of one or a plurality of implants comprising osseous anchoring means such as, for example, a screw or one or a plurality of hooks. The implants thus comprise fixation means capable of receiving the bar before or in the course of the surgical procedure, then fixing said bar to the implant.

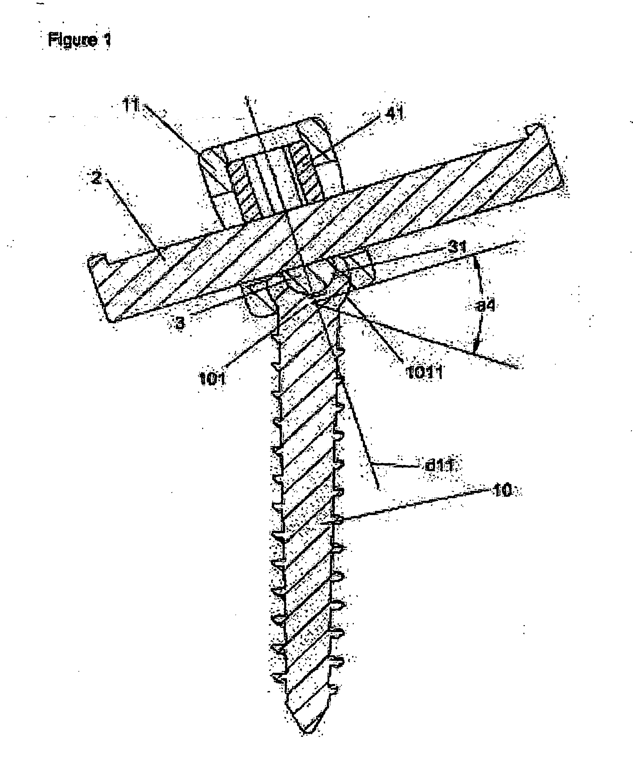

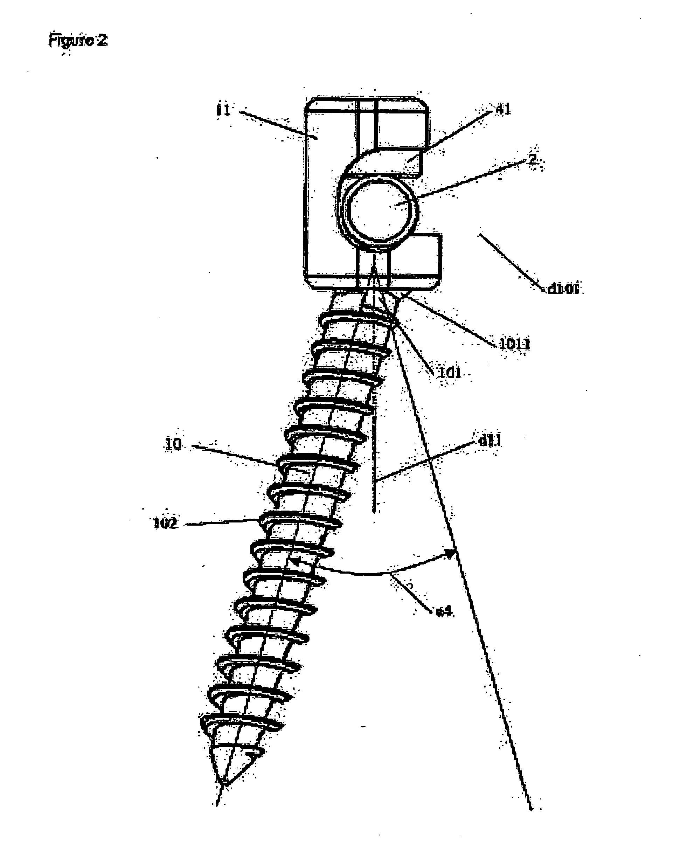

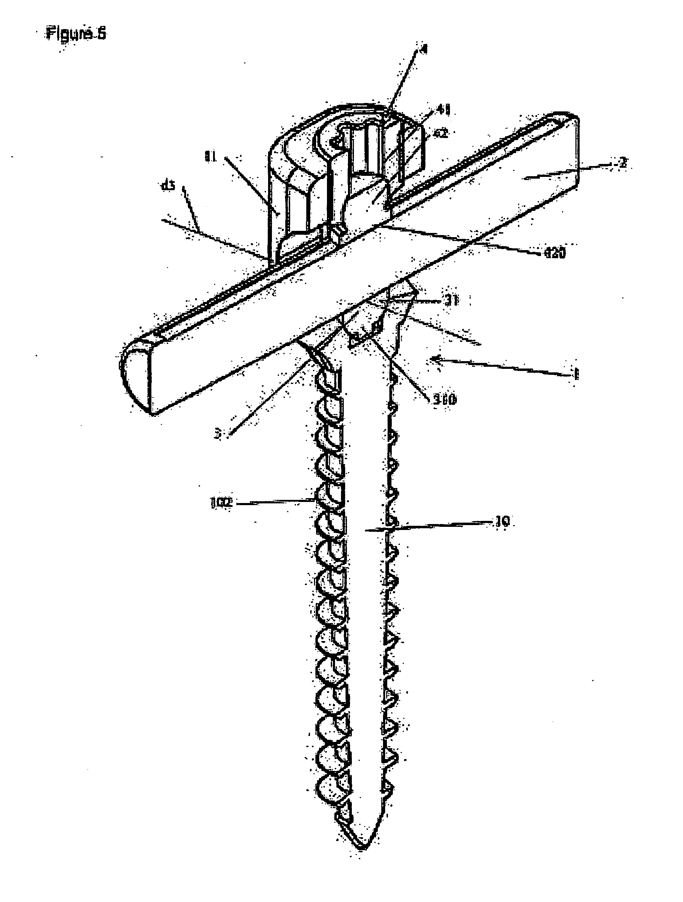

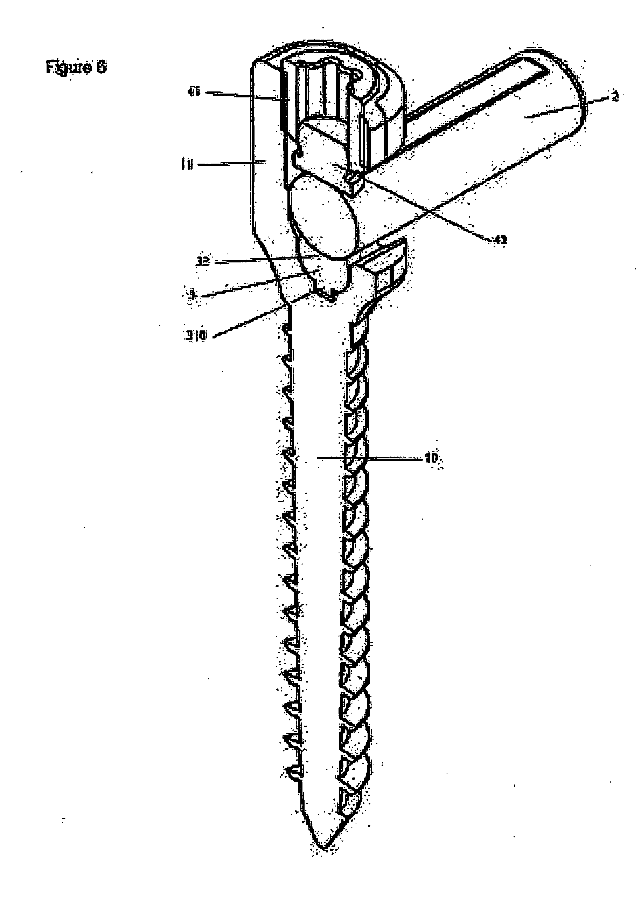

In one example represented in Figures 1 to 4 and in the examples represented in figures 5 and 6, said anchoring means are realized by an elongated part having a thread and capable of being screwed into the osseous material, for example at the pedicle or body of a vertebra or into the sacrum.

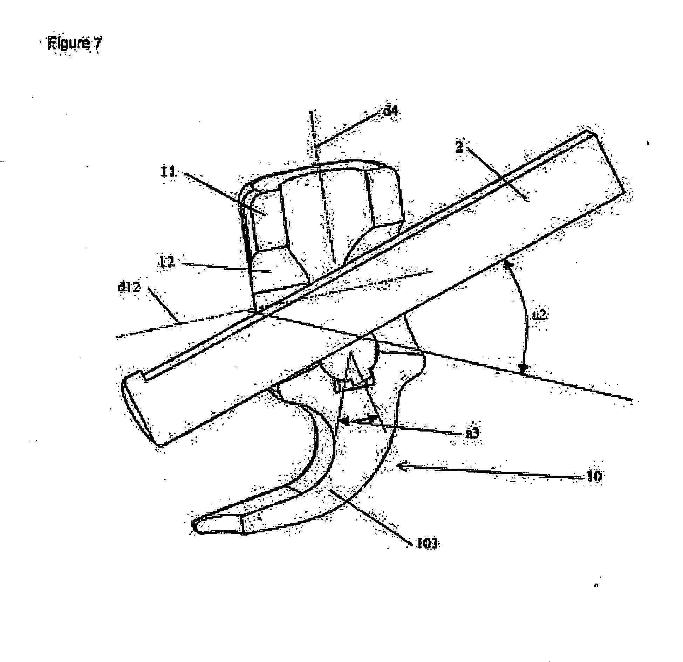

In one example represented in Figure 7, said osseous anchoring means comprise a hook intended for being engaged to a formal irregularity in the osseous element, such as a pedicle or a vertebral or sacral protrusion, or a transverse apophysis.

In the course of the surgical procedure, when an implant is anchored in an osseous element and that a bar is to be fixed to it, the position in which the bar can be introduced does not always correspond to that, which would be the easiest for assembly to the fixation means of the implant. Said position can be restricted, for example, by the anatomical environment or by the fact that said bar is already assembled in another implant.

Moreover, when the bar is inserted into the fixation means, if the latter are not properly aligned with the axis of the bar, the arrangement of the contact or fixation surfaces can be the cause of poor fixation, not being sufficiently rigid or secure.

In particular, if the bar must be forced at the time of fixation to be adapted to the relative position of the implants, said force can result in residual stresses in the structure of the osteosynthesis device. Such persisting stresses can consequently impair the patient's daily comfort or perturb or change the desired effect of the device.

In order that the fixation means can be better adjusted to the position of the bar, the fixation means according to the invention comprise at least one element having a certain moveability. Said element can be adjusted by a rotation around one or a plurality of axes not merged with the longitudinal axis of the bar and, for example, perpendicular to said longitudinal axis. Said rotation can be executed according to a determined clearance capable of comprising maximal angular positions or according to predetermined angular positions or capable of being unlimited, that is completely free. According to the embodiments, said fixation means can comprise an element moving along one or a plurality of axes or they may comprise a plurality of elements themselves moving along one or a plurality of axes.

The bar can thus be assembled in a plurality of angular positions relative to the anchoring means or to the osseous element. These variations in angular position can particularly comprise an adjustable tilt relative to the osseous surface where the implant is anchored or a rotation around an axis extending from said osseous surface or a combination of the two.

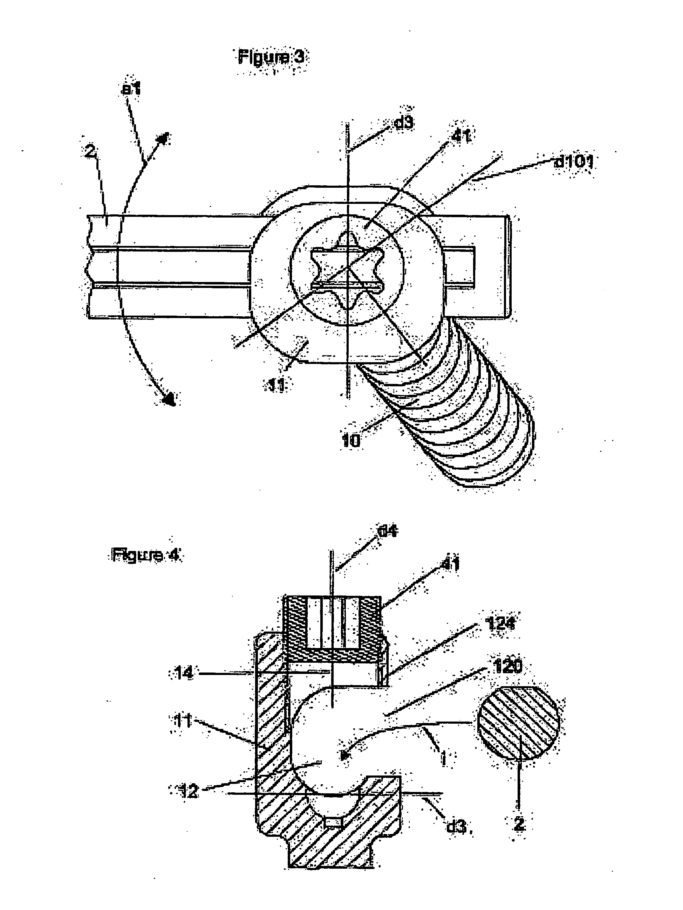

In the examples represented herein, the implant (1) is rigidly connected to the bar (2) by fixation means comprising a channel (12) housed in one part of the implant, said part being designated as the fixation head (11). The channel (12) can exhibit the shape of a channel opening at its two ends and open on one of its sides. Such a lateral opening (120) thus enables introduction (i; Figure 4) of the bar (2) through the side of the channel without the necessity of having to thread the bar through an end.

Once inserted into the channel (12), fixation of the bar is assured by clamping means (4) that are supported by means of at least one side of said bar to make contact and block it against one wall, called the support wall, of the channel (12). Said clamping means comprise, for example, a clamping screw (41) mounted in a drilling (14) borne by one part of the fixation head (11) constituting one edge (124) of the lateral opening (120) of the channel (12). Said clamping screw (41) has an external threading that co-operates with an internal threading of this drilling (14) to displace the screw (41) along a clamping axis (d4) and bring it into contact against the bar, thus clamping clamping screw (41) can comprise formal irregularities, for example an internal mark, enabling the use of a clamping tool to realize blocking the bar (2). The bar can advantageously have one or a plurality of flats upon its external surface to enable obtaining a flat contact surface with the clamping screw (41) and thus enhanced reliability of blocking than with a punctal or linear contact.

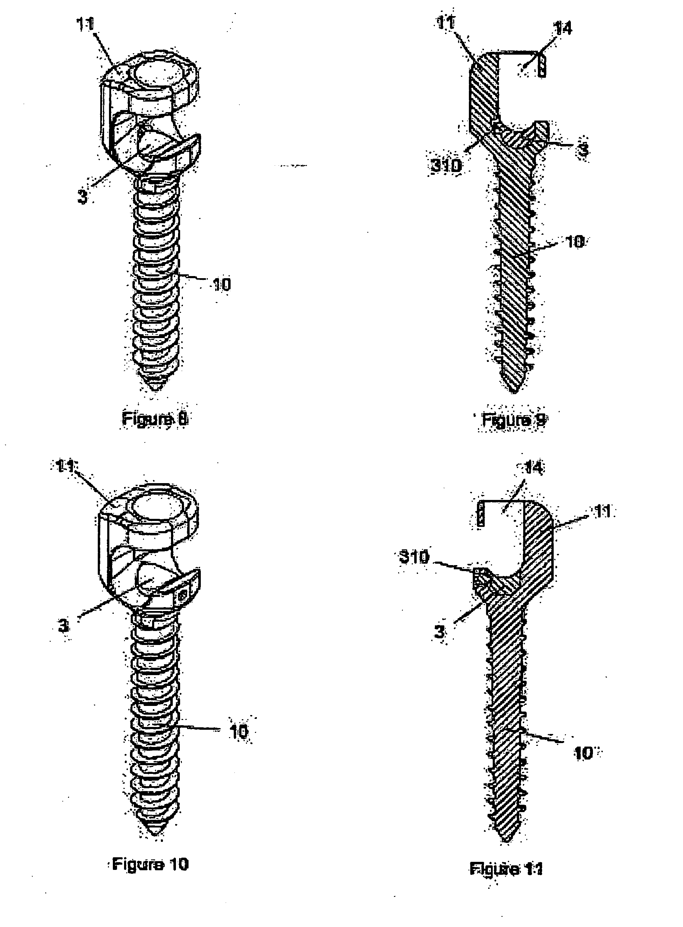

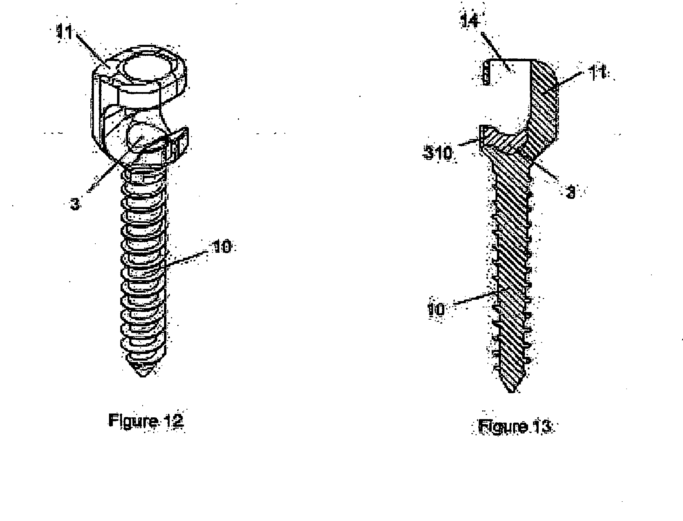

In the examples represented in Figures 4 to 13, the channel (12) has a support wall providing an element, called a moving baseplate (3), said baseplate being moving relative to the fixation head (11). Said moving baseplate (3) has a part (31) in form of a sphere portion, leaning by a complementary contact in a housing formed in the wall of the channel (12). By virtue of said spherical contact, the moving baseplate (3) disposes of a certain freedom of rotation around the center of its spherical part (31). Said moving baseplate has in particular a certain clearance (a3) in rotation around an axis (d3) substantially perpendicular to the longitudinal axis (d12) of the channel and to the direction of support of the clamping means. On its face, called support face (32), at the contact with the bar, the moving baseplate has a shape complementary to the external surface of said bar, for example in the form of a cylindrical portion, that provides a good contact surface when clamping.

On its part (31) in form of a sphere portion the moving baseplate (3) can have one or a plurality of formal irregularities (310) co-operating with one or a plurality of formal irregularities of the housing of the fixation head (11) to form a stop limiting the clearance in rotation of the moving baseplate. Said formal irregularities (310) can be, for example, a pin protuding from the moving baseplate and co-operating with a larger dimensioned cavity formed on the complementary contact surface. Said stop, for example, allows avoiding excessive turning of the moving baseplate and assuring that it properly presents its support face facing the bar.

With regard to the bar (2), the inside surface of the channel (12) is of sufficient dimensions to enable the bar a certain clearance (a2) in rotation around one or a plurality of axes not parallel to the longitudinal axis of the bar or, in particular, perpendicular to this longitudinal axis.

At its end on the bar side, the clamping screw (41) exhibits a moving element, called the support head (42), articulated by a ball and socket connection. The screwing of the clamping screw (41) provokes the leaning of said support head (42) on the flat of the bar (2) through one moving clamping face (420). Said ball and socket connection allows a certain clearance of the support head (42) relative to the clamping screw (41) in rotation around the center of said ball and socket connection. By a rotation around at least one axis parallel to the axis of rotation (d3) of the moving baseplate (3), the moving support face (420) can thus be permanently adjusted to the position of the bar and the moving baseplate. Said ball and socket connection also enables the support face to remain in contact with the bar without sliding over it, which avoids deterioration of the surfaces in contact, assures the blockage, and reduces the risk of residual stresses. The examples shown in the figures 4 to 13 may of course be combined with the examples shown in figures 1 to 3, because to articulation of the osseous anchoring means relative to the head is fully compatible with the ball and socket connection and/or with the stops of the moving baseplate and so one. The various possible combinations of the examples shown with the embodiment in which the osseous anchoring is articulated relative to the fixation head should thus be considered as part of the invention.

Thus, it can be understood, that the bar can be inserted and blocked in different angular positions inside the channel (12), while providing a flat contact surface both with the clamping means and with the wall of the channel by means of the moving baseplate (3). Said polyaxial angular clearance thus allows inserting the bar more easily and obtaining a clamping of the bar in its most natural position relative to the implants, which reduces or eliminates the stresses that could subsist in the device after clamping. Furthermore, the clamping forces concur directly with blocking without necessarily opposing the rigidity of the bar and the reliability of the blocking is thus improved.

In an example represented in Figures 1 to 3, the fixation head (11) is moving relative to the osseous anchoring part (10) according to an articulation enabling freedom in rotation around at least two axes not merged with the longitudinal axis of the bar.

This articulation is realized by a complementary spherical contact between the fixation head (11) and the end of the osseous anchorage part (10) remote from the osseous element, said end being designated as the rotation head (101). The rotation head comprises a part (1011) in form of a hemispherical portion widening in the direction of the fixation head (11); that is, by moving away from the osseous element. Said hemispherical portion(1011) is retained on the inside of the fixation head (11) by a complementary contact in a housing formed in said fixation head and narrowing towards the osseous anchorage part. Said housing communicates with the channel (12) where it opens in its part situated opposite to the clamping means. The spherical nature of these contact surfaces thus enable a rotation of the fixation head and the osseous anchorage part relative to each other, in rotation about the center of said surface (1011) in a hemispherical form.

Said rotations enable, in particular, unlimited clearance (a1) of the fixation head relative to the osseous anchorage part, in rotation around an axis (d11), called the axis of rotation of the head, non parallel, indeed even perpendicular to the longitudinal axis of the bar or of the channel and passing through the center of the hemisphere (1011) of the rotation head (101).

These rotations also enable a certain clearance (a4) of the fixation head relative to the osseous anchorage part, in rotation around an axis substantially perpendicular to the axis (d11) of rotation of the head and passing through the center of the hemisphere (1011) of the rotation head (101).

In this example, a moving base (3) similar to that hereinbefore described is borne by the rotation head (101) in a housing formed on the face opposite to the bar.

At the time of clamping of the clamping means, the clamping screw (41) co-operates by its threading with the drilling (14) of the fixation head (11) to lean on the bar (2). The bar leans on the moving baseplate (3). The moving baseplate (3) leans on the rotation head (101), which is retained by the housing of the fixation head (11). Clamping of the clamping means leads to a leaning of whole these surfaces among themselves, which produces a blocking of the set of these parts relative to each other.

It is well understood that in this manner an implant is provided, whose fixation head, prior to blocking of the clamping means, is moving relative to the osseous anchoring part, while being fixed to the bar after said blockage. The fixation head (11) can thus be tilted according to a certain clearance relative to the exterior surface of the osseous element and can pivot freely around an axis extending from said osseous surface.

Once the implant is anchored in the osseous element, it is thus still possible to adjust the position of the fixation head in order to enable the bar to keep or to resume its shape, which reduces the risks of residual stresses and permits easy introduction of the bar into diverse positions of this bar and implants. Once the bar is introduced and the whole device assembled, it is thus possible to block said positions by virtue of the clamping means. As clamping can be achieved in the most natural position of the pin, the clamping forces are concentrated at the best on the blocking reliability. In particular, these clamping forces do not risk, or the risk thereof is minimal, introducing in the device residual stresses or movements relative to the position selected by the surgeon.

In this example, the clamping means (4) need not comprise a support head (42) on the ball and socket connection, in particular if the tilting of the fixation head (11) is sufficiently close to that of the bar (2) to assure a planar contact between the clamping screw (41) and the flat of the bar.

It should be clear to the those skilled in the art that the present invention enables embodiments in many specific forms without moving it away from the field of application of the invention as claimed. Consequently, the present embodiments must be considered illustrative, but can be modified in the field defined by the scope of the attached claims and the invention should not be limited to the details provided above.

## Claims

1. An implant (1) for osseous anchoring comprising fixation means capable of receiving and fixing at least one bar (2), in particular of osteosynthesis, the implant (1) comprising on the one hand osseous anchoring means (10) and on the other hand a fixation head (11) bearing the fixation means, the fixation head being traversed by at least one channel (12) receiving the bar (2) and comprising clamping means (4) capable of clamping the bar against one inside wall, called support wall (32), the clamping means (4) and said support wall (32) enabling obtaining, prior to blocking of the fixation, a determined clearance in rotation around at least one first axis (d3) not parallel to the longitudinal axis of the bar, the clamping means comprising a face at the contact with the bar, called the moving clamping face (420), the moving clamping face (420) of the clamping means (4) being borne by a support head (42) articulated at the end of the clamping means (4) by a bail and socket connection, the implant (1) being **characterized in that** the channel (12) has the form of an open channel having an aperture (120) opening onto one of the lateral faces of the fixation head (11), one edge (124) of said aperture bearing the clamping means (4), the aperture (120) of the channel and the position of the clamping means thus enabling the introduction (i) of the bar by the lateral route, **in that** the clamping means (4) and said support wall (32) enable obtaining, prior to blocking of the fixation, a determined clearance in rotation around at least one second axis (d4) not parallel to the first axis (d3) and not parallel to the longitudinal axis of the bar and **in that** the fixation head (11) is mobile relative to the osseous anchoring part (10) according to an articulation enabling freedom in rotation around at least two axes not parallel to the longitudinal axis of the bar.

2. An implant according to claim 1, wherein the articulation between the fixation head (11) and the osseous anchoring part providing freedom of rotation consist in a rotation head (101) at an extremity of the osseous anchoring part (10), the fixation head being movable relating to this rotation head, at least prior the blocking of the clamping means.

3. An implant according to claim 2, wherein the fixation head (11) presents relating to the rotation head (101) on the one hand an unlimited clearance (a1) in rotation around an axis, called head rotation axis (d11), not parallel to the channel axis (12) or to the longitudinal axis of the bar.

4. An implant according to claim 3, wherein the fixation head (11) presents relating to the rotation head (101) a determined clearance (a4) in rotation around an axis, called head tilting axis (d101), not parallel to the head rotation axis (d11).

5. An implant according to anyone of claims 2 to 4, wherein the clamping means (4) bear the bar (2) on a moving baseplate (3) comprising sphere portion part (31), this sphere portion bearing in a complementary housing formed in the rotation head (101), the rotation head itself comprising an external rotation surface (1011) in form of a half sphere portion bellmouthed toward the fixation head (11), this external rotation surface (1011) being retained in a housing having a complementary shape prepared in the fixation head (11), the clamping of the clamping means (4) inside the fixation head leading thus to a clamping and stopping of all of these complementary surfaces being in contact.

6. An implant according to anyone of claims 1 to 5, wherein the channel (12) opens at each side of the fixation head (11) through apertures whose disposition and dimensions enable to the bar a determined clearance (a2) inside the channel (12) in rotation at least around one axis that is substantially perpendicular to the axis (d12) of the channel and to the support direction (d4) of the clamping means.

7. An implant according to anyone of claims 1 to 6, wherein the support wall comprises an element, called the moving baseplate (3), having a clearance (a3) in rotation at least around one axis (d3) not parallel to the axis (d12) of the channel (12) and to the support direction (d4) of the clamping means on the bar (2), the clamping means comprising a face at the contact with the bar, called moving clamping face (420), having a determined clearance in rotation around one axis substantially parallel to the axis (d3) of rotation of the moving baseplate.

8. An implant according to claim 7, wherein the moving baseplate (3) has a part in form of a sphere portion (31) leaning by a complementary contact in a housing formed in the fixation head (11).

9. An implant according to anyone of claims 1 to 8, wherein the moving baseplate (3) is in contact with the bar (2) by a support face (32) 10 having a shape substantially complementary to the exterior shape of the bar, the moving baseplate having at least one formal irregularity (310) co-operating with a formal irregularity (130) borne by the fixation head (11) to form a stop holding the support face (32) of the baseplate turned towards the bar (2).

10. An implant according to Claim 9, wherein the formal irregularity (310) is a pin formed on the underside, the rear or on the front of the moving baseplate (3), co-operating with a cavity with a greater dimension borne by the fixation head.

11. An implant according to Claim 9, wherein the formal irregularity (310) is a cylindrical drilling formed on the front of the moving baseplate (3) and co-operating with the cylindrical head of a pin held in the drilling formed in the fixation head (11).

12. An implant according to anyone of claims 1 to 11, wherein the clamping means (4) comprise a clamping screw (41) inserted into a drilling (14) traversing one edge (124) of the channel opening and co-operating with said drilling to realize a support upon the bar (2).

13. An implant according to anyone of claims 1 to 12, wherein the osseous anchoring means comprise either a threaded part (102) capable of anchoring in an osseous element by co-operation with the osseous material of said osseous element, or a protruding part (103) capable of anchoring at the surface of an osseous element by co-operating with at least one formal irregularity of said same surface.

14. An implant according to claim 13, wherein the osseous anchoring means are realized by an elongated and threaded part, the axis (d12) of the channel (12) being substantially perpendicular to the longitudinal axis of the osseous anchoring means.

15. An implant according to anyone of claims 1 to 14, wherein the channel (d12) has a shape comprising two head to tail truncated cones facing each other with their minor baseplates and joined to each other directly or by means of a cylindrical part.

16. An implant according to anyone of Claims 1 to 15, wherein the bar (2) bears one or a plurality of flats on its external surface, providing a flat contact surface with the support head (42).

## Patentansprüche

1. Implantat (1) zur Knochenverankerung, aufweisend Fixierungsmittel, die zum Aufnehmen und Fixieren mindestens eines Stabes (2), insbesondere zur Osteosynthese, geeignet sind, wobei das Implantat (1) aufweist: einerseits Knochenverankerungsmittel (10) und andererseits einen Fixierungskopf (11), der die Fixierungsmittel trägt, wobei der Fixierungskopf von mindestens einem Kanal (12) durchquert wird, der den Stab (2) aufnimmt und Klemm-Mittel (4) aufweist, die zum Klemmen des Stabes gegen eine Innenwand geeignet sind, die als Stützwand (32) bezeichnet wird, wobei die Klemm-Mittel (4) und die Stützwand (32) vor dem Blockieren der Fixierung das Erhalten eines bestimmten Rotationsspielraums um mindestens eine erste Achse (d3) ermöglichen, die nicht parallel zu der Längsachse des Stabes ist, wobei die Klemm-Mittel an der Kontaktstelle mit dem Stab eine Fläche aufweisen, die als Bewegungs-Klemmfläche (420) bezeichnet wird, wobei die Bewegungs-Klemmfläche (420) der Klemm-Mittel (4) von einem Stützkopf (42) getragen wird, der an dem Ende der Klemm-Mittel (4) mittels einer Kugelgelenkverbindung gelenkig gelagert ist, wobei das Implantat (1) **dadurch gekennzeichnet ist, dass** der Kanal (12) die Form eines offenen Kanals mit einer Öffnung (120) aufweist, die sich zu einer der Seitenflächen des Fixierungskopfes (11) hin öffnet, wobei ein Rand (124) der Öffnung die Klemm-Mittel (4) trägt, wobei die Öffnung (120) des Kanals und die Position der Klemm-Mittel somit das Einführen (i) des Stabes über einen seitlichen Weg ermöglichen, dass die Klemm-Mittel (4) und die Stützwand (32) vor dem Blockieren der Fixierung das Erhalten eines bestimmten Rotationsspielraums um mindestens eine zweite Achse (d4) ermöglichen, die nicht parallel zu der ersten Achse (d3) und nicht parallel zu der Längsachse des Stabes ist, und dass der Fixierungskopf (11) relativ zu dem Knochenverankerungsteil (10) gemäß einer Gelenkverbindung mobil ist, die einen Rotationsspielraum um mindestens zwei Achsen ermöglicht, die nicht parallel zu der Längsachse des Stabes sind.

2. Implantat gemäß Anspruch 1, wobei die Gelenkverbindung zwischen dem Fixierungskopf (11) und dem Knochenverankerungsteil, die einen Rotationsspielraum bereitstellt, aus einem Rotationskopf (101) an einem Ende des Knochenverankerungsteils (10) besteht, wobei der Fixierungskopf relativ zu dem Rotationskopf zumindest vor dem Blockieren der Klemm-Mittel bewegbar ist.

3. Implantat gemäß Anspruch 2, wobei der Fixierungskopf (11) relativ zu dem Rotationskopf (101) einerseits einen unbegrenzten Rotationsspielraum (a1) um eine Achse aufweist, die als Kopf-Rotationsachse (d11) bezeichnet wird, die nicht parallel zu der Kanal-Achse (12) oder zu der Längsachse des Stabes ist.

4. Implantat gemäß Anspruch 3, wobei der Fixierungskopf (11) relativ zu dem Rotationskopf (101) einen bestimmten Rotationsspielraum (a4) um eine Achse aufweist, die als Kopf-Schwenkachse (d101) bezeichnet wird, die nicht parallel zu der Kopf-Rotationsachse (d11) ist.

5. Implantat gemäß einem der Ansprüche 2 bis 4, wobei die Klemm-Mittel (4) den Stab (2) auf einer sich bewegenden Basisplatte (3) tragen, die einen Kugelabschnittsteil (31) aufweist, wobei der Kugelabschnitt in einem komplementären Gehäuse gelagert ist, das in dem Rotationskopf (101) ausgebildet ist, wobei der Rotationskopf selbst eine äußere Rotationsfläche (1011) in Form eines Halbkugelabschnitts aufweist, der in Richtung zu dem Fixierungskopf (11) trichterförmig ausgebildet ist, wobei die äußere Rotationsfläche (1011) in einem Gehäuse mit einer komplementären Form, das in dem Fixierungskopf (11) bereitgestellt ist, gehalten wird, wobei das Klemmen der Klemm-Mittel (4) in dem Fixierungskopf somit zu einem Festklemmen und Stoppen aller komplementärer in Kontakt befindlicher Flächen führt.

6. Implantat gemäß einem der Ansprüche 1 bis 5, wobei sich der Kanal (12) an jeder Seite des Fixierungskopfes (11) durch Öffnungen hindurch öffnet, deren Anordnung und Abmessungen dem Stab einen bestimmten Spielraum (a2) innerhalb des Kanals (12) beim Drehen um mindestens eine Achse ermöglichen, die im Wesentlichen senkrecht zu der Achse (d12) des Kanals und zu der Stützrichtung (d4) der Klemm-Mittel ist.

7. Implantat gemäß einem der Ansprüche 1 bis 6, wobei die Stützwand ein Element aufweist, das als Bewegungs-Basisplatte (3) bezeichnet wird, mit einem Rotationsspielraum (a3) mindestens um eine Achse (d3), die nicht parallel zu der Achse (d12) des Kanals (12) und zu der Stützrichtung (d4) der Klemm-Mittel auf dem Stab (2) ist, wobei die Klemm-Mittel eine Fläche an der Kontaktstelle mit dem Stab aufweisen, die als Bewegungs-Klemmfläche (420) bezeichnet wird, mit einem bestimmten Rotationsspielraum um eine Achse, die im Wesentlichen parallel zu der Rotationsachse (d3) der Bewegungs-Basisplatte ist.

8. Implantat gemäß Anspruch 7, wobei die Bewegungs-Basisplatte (3) einen Teil in Form eines Kugelabschnitts (31) aufweist, der durch einen komplementären Kontakt in einem Gehäuse gestützt wird, das in dem Fixierungskopf (11) ausgebildet ist.

9. Implantat gemäß einem der Ansprüche 1 bis 8, wobei die Bewegungs-Basisplatte (3) durch eine Stützfläche (32) in Kontakt mit dem Stab (2) ist, die eine Form aufweist, die im Wesentlichen komplementär zu der äußeren Form des Stabes ist, wobei die Bewegungs-Basisplatte mindestens eine Formunebenheit (310) aufweist, die mit einer Formunebenheit (130) zusammenwirkt, die von dem Fixierungskopf (11) getragen wird, zum Ausbilden eines Anschlages, der die Stützfläche (32) der Basisplatte in Richtung zu der Seite des Stabes (2) gedreht hält.

10. Implantat gemäß Anspruch 9, wobei die Formunebenheit (310) ein Stift ist, der an der Unterseite, der Rückseite oder der Vorderseite der Bewegungs-Basisplatte (3) ausgebildet ist, der mit einer Aussparung mit einer größeren Abmessung zusammenwirkt, die von dem Fixierungskopf getragen wird.

11. Implantat gemäß Anspruch 9, wobei die Formunebenheit (210) eine zylinderförmige Bohrung ist, die an der Vorderseite der Bewegungs-Basisplatte (3) ausgebildet ist und mit dem zylinderförmigen Kopf eines Stiftes zusammenwirkt, der in der Bohrung gehalten wird, die in dem Fixierungskopf (11) ausgebildet ist.

12. Implantat gemäß einem der Ansprüche 1 bis 11, wobei die Klemm-Mittel (4) eine Klemmschraube (41) aufweisen, die in eine Bohrung (14) eingesetzt ist, die einen Rand (124) der Kanalöffnung durchquert und derart mit der Bohrung zusammenwirkt, dass ein Abstützen an dem Stab (2) realisiert wird.

13. Implantat gemäß einem der Ansprüche 1 bis 12, wobei die Knochenverankerungsmittel aufweisen: entweder einen Gewindeteil (102), der zum Verankern in einem Knochenelement durch Zusammenwirken mit dem Knochenmaterial des Knochenelements geeignet ist, oder einen hervorstehenden Teil (103), der zum Verankern an der Oberfläche eines Knochenelements durch Zusammenwirken mit mindestens einer Formunebenheit derselben Oberfläche geeignet ist.

14. Implantat gemäß Anspruch 13, wobei die Knochenverankerungsmittel durch einen länglichen und mit einem Gewinde versehenen Teil ausgebildet sind, wobei die Achse (d12) des Kanals (12) im Wesentlichen senkrecht zu der Längsachse der Knochenverankerungsmittel ist.

15. Implantat gemäß einem der Ansprüche 1 bis 14, wobei der Kanal (d12) eine Form aufweist, die zwei Kopfzu-Ende-Kegelstümpfe aufweist, die einander mit ihrer kleineren Basisplatte zugewandt sind und direkt oder mittels eines zylinderförmigen Teils miteinander verbunden sind.

16. Implantat gemäß einem der Ansprüche 1 bis 15, wobei der Stab (2) eine oder eine Mehrzahl von Abflachungen an seiner Außenfläche trägt, von denen eine flache Kontaktfläche mit dem Stützkopf (42) bereitgestellt wird.

## Revendications

1. Implant (1) pour un ancrage osseux comprenant des moyens de fixation pouvant recevoir et fixer au moins une barre (2), en particulier d'ostéosynthèse, l'implant (1) comprenant d'une part des moyens d'ancrage osseux (10) et d'autre part une tête de fixation (11) supportant les moyens de fixation, la tête de fixation étant traversée par au moins un canal (12) recevant la barre (2) et comprenant des moyens de serrage (4) pouvant serrer la barre contre une paroi interne, appelée paroi de support (32), les moyens de serrage (4) et ladite paroi de support (32) permettant d'obtenir, avant de bloquer la fixation, un jeu déterminé en rotation autour d'au moins un premier axe (d3) non parallèle à l'axe longitudinal de la barre, les moyens de serrage comprenant une face au niveau du contact avec la barre, appelée face de serrage mobile (420), la face de serrage mobile (420) des moyens de serrage (4) étant supportée par une tête de support (42) articulée au niveau de l'extrémité des moyens de serrage (4) par une liaison rotule, l'implant (1) étant **caractérisé en ce que** le canal (12) a la forme d'un canal ouvert ayant une ouverture (120) s'ouvrant sur l'une des faces latérales de la tête de fixation (11), un bord (124) de ladite ouverture supportant les moyens de serrage (4), l'ouverture (120) du canal et la position des moyens de serrage permettant ainsi l'introduction (i) de la barre par la voie latérale, **en ce que** les moyens de serrage (4) et ladite paroi de support (32) permettent d'obtenir, avant de bloquer la fixation, un jeu déterminé en rotation autour d'au moins un second axe (d4) non parallèle au premier axe (d3) et non parallèle à l'axe longitudinal de la barre et **en ce que** la tête de fixation (11) est mobile par rapport à la partie d'ancrage osseux (10), selon une articulation permettant une liberté en rotation autour d'au moins deux axes non parallèles à l'axe longitudinal de la barre.

2. Implant selon la revendication 1, dans lequel l'articulation entre la tête de fixation (11) et la partie d'ancrage osseux fournissant la liberté de rotation se compose d'une tête de rotation (101) à une extrémité de la partie d'ancrage osseux (10), la tête de fixation étant mobile par rapport à cette tête de rotation, au moins avant le blocage des moyens de serrage.

3. Implant selon la revendication 2, dans lequel la tête de fixation (11) présente en ce qui concerne la tête de rotation (101), d'une part, un jeu illimité (a1) en rotation autour d'un axe, appelé axe de rotation de tête (d11), non parallèle à l'axe de canal (12) ou à l'axe longitudinal de la barre.

4. Implant selon la revendication 3, dans lequel la tête de fixation (11) présente en ce qui concerne la tête de rotation (101), un jeu déterminé (a4) en rotation autour d'un axe, appelé axe d'inclinaison de tête (d101), non parallèle à l'axe de rotation de tête (d11).

5. Implant selon l'une quelconque des revendications 2 à 4, dans lequel les moyens de serrage (4) supportent la barre (2) sur une plaque de base mobile (3) comprenant une partie de portion de sphère (31), cette portion de sphère étant supportée dans un boîtier complémentaire formé dans la tête de rotation (101), la tête de rotation elle-même comprenant une surface de rotation externe (1011) sous la forme d'une portion de demi-sphère en corolle vers la tête de fixation (11), cette surface de rotation externe (1011) étant retenue dans un boîtier ayant une forme complémentaire préparée dans la tête de fixation (11), le serrage des moyens de serrage (4) à l'intérieur de la tête de fixation conduisant ainsi à un serrage et un arrêt de toutes ces surfaces complémentaires qui sont en contact.

6. Implant selon l'une quelconque des revendications 1 à 5, dans lequel le canal (12) s'ouvre de chaque côté de la tête de fixation (11) par des ouvertures dont la disposition et les dimensions permettent à la barre, un jeu déterminé (a2) à l'intérieur du canal (12) en rotation au moins autour d'un axe qui est sensiblement perpendiculaire à l'axe (d12) du canal et à la direction de support (d4) des moyens de serrage.

7. Implant selon l'une quelconque des revendications 1 à 6, dans lequel la paroi de support comprend un élément, appelé plaque de base mobile (3), ayant un jeu (a3) en rotation au moins autour d'un axe (d3) non parallèle à l'axe (d12) du canal (12) et à la direction de support (d4) des moyens de serrage sur la barre (2), les moyens de serrage comprenant une face au niveau du contact avec la barre, appelée face de serrage mobile (420), ayant un jeu déterminé en rotation autour d'un axe sensiblement parallèle à l'axe (d3) de rotation de la plaque de base mobile.

8. Implant selon la revendication 7, dans lequel la plaque de base mobile (3) a une partie ayant la forme d'une portion de sphère (31) qui s'incline par un contact complémentaire dans un boîtier formé dans la tête de fixation (11).

9. Implant selon l'une quelconque des revendications 1 à 8, dans lequel la plaque de base mobile (3) est en contact avec la barre (2) par une face de support (32) ayant une forme sensiblement complémentaire à la forme extérieure de la barre, la plaque de base mobile ayant au moins une irrégularité formelle (310) coopérant avec une irrégularité formelle (130) supportée par la tête de fixation (11) afin de former une butée maintenant la face de support (32) de la plaque de base tournée vers le côté de la barre (2).

10. Implant selon la revendication 9, dans lequel l'irrégularité formelle (310) est une broche formée sur la face inférieure, l'arrière ou l'avant de la plaque de base mobile (3), coopérant avec une cavité avec une dimension plus importante supportée par la tête de fixation.

11. Implant selon la revendication 9, dans lequel l'irrégularité formelle (310) est une perforation cylindrique formée sur l'avant de la plaque de base mobile (3) et coopérant avec la tête cylindrique d'une broche maintenue dans la perforation formée dans la tête de fixation (11).

12. Implant selon l'une quelconque des revendications 1 à 11, dans lequel les moyens de serrage (4) comprennent une vis de serrage (41) insérée dans une perforation (14) traversant un bord (124) de l'ouverture de canal et coopérant avec ladite perforation pour réaliser un support sur la barre (2).

13. Implant selon l'une quelconque des revendications 1 à 12, dans lequel les moyens d'ancrage osseux comprennent une partie filetée (102) pouvant s'ancrer dans un élément osseux par la coopération avec le matériau osseux dudit élément osseux ou une partie en saillie (103) pouvant s'ancrer dans la surface d'un élément osseux en coopérant avec au moins une irrégularité formelle de ladite même surface.

14. Implant selon la revendication 13, dans lequel les moyens d'ancrage osseux sont réalisés par une partie allongée et filetée, l'axe (d12) du canal (12) étant sensiblement perpendiculaire à l'axe longitudinal des moyens d'ancrage osseux.

15. Implant selon l'une quelconque des revendications 1 à 14, dans lequel le canal (d12) a une forme comprenant deux cônes tronqués tête bèche se faisant face avec leurs plaques de base mineures et assemblés directement ou au moyen d'une partie cylindrique.

16. Implant selon l'une quelconque des revendications 1 à 15, dans lequel la barre (2) supporte un ou une pluralité de plats sur sa surface externe, fournissant une surface de contact plate avec la tête de support (42).
